# EUROPEAN PATENT APPLICATION

(11) **EP 4 763 193 A1**
(43) Date of publication of application: **24.06.2026**
(21) Application number: 24220745.4
(22) Date of filing: 17.12.2024
(51) Int. Cl.: A61K 9/10, A61K 31/685, A61K 31/719, A61K 36/889, A61K 47/36, A61P 13/08, A61P 15/00

(54) **STABILIZED FORMULATION OF SERENOA REPENS**

(71) Applicant: FB Dermo srl, 20149 Milano (IT)
(72) Inventor: BANFI, Fabio, 20145 Milano (IT)
(74) Representative: Manfredi, Irene

(57) **Abstract**

The present invention discloses a composition for the treatment of androgen-dependent disorders, such as androgenetic alopecia, benign prostatic hyperplasia, and prostate cancer, comprising a *Serenoa repens* extract as an active ingredient, and excipients, suitable for forming a stable, homogeneous matrix in the form of an emulsion when mixed with the *Serenoa repens* extract, appropriately dosed, in the presence of water; said emulsion is also suitable to be made into a powder by spray drying, thereby maintaining the biological activity of the composition.

## Description

### BACKGROUND OF THE INVENTION

The present invention relates to a composition for the treatment of androgen-dependent disorders, such as androgenetic alopecia, benign prostatic hyperplasia, and prostate cancer, comprising a *Serenoa repens* extract as an active ingredient, and excipients, suitable for forming a stable and homogeneous matrix in the form of an emulsion when mixed with the *Serenoa repens* extract, appropriately dosed, in the presence of water; said emulsion is also suitable to be made into powder form by spray drying, thereby maintaining the biological activity of the composition.

### PRIOR ART

*Serenoa repens* (Serenoa repens, syn. Sabal serrulata), also known as American dwarf palm (saw palmetto), is a small palm tree native to the eastern United States. The biologically active substances are contained in the fruits of this plant, which once ripe consist of globular berries of a bluish-black colour, which are desiccated before being processed.

*Serenoa repens* plant fruit extracts (hereinafter also referred to as "Serenoa extracts", for brevity) are known for their anti-androgenic activity. Indeed, Serenoa acts on a dual level, firstly by blocking the enzyme 5α-reductase, which is responsible for the transformation of testosterone into the active metabolite dihydrotestosterone (DHT) thereof, and secondly by preventing DHT interaction with its receptor (androgen receptor, AR); hence the use of Serenoa-based drugs in the treatment of androgen-dependent disorders, such as benign prostatic hypertrophy or hyperplasia, prostate cancer, androgenetic alopecia, chronic pelvic pain, inflammation of the urinary tract, especially the bladder, diuresis disorders, decreased sexual desire, hormonal imbalances.

Typically, drugs and food supplements based on *Serenoa repens,* for the treatment of the disorders listed above, include Serenoa lipid extracts formulated with suitable excipients. A Serenoa lipid extract can be obtained by any of the commonly known extraction processes, typically by extraction in hexane.

In general, lipid extraction of Serenoa has a weight yield of 10% from the plant material and the extract is composed of about 85% free fatty acids and about 15% a mixture of ethyl and methyl esters of said acids, long-chain saturated and unsaturated alcohols in free and sterile form with the above acids and with triterpene sterols and alcohols present in free and sterile form. The main free fatty acids are lauric acid (about 30%), oleic acid (about 30%), myristic acid (about 10%), and palmitic acid (about 10%). Fatty acids and phytosterols are considered to be the bioactive components of Serenoa extract.

The Serenoa lipid extract has an oily liquid form.

Medicines in liquid form are known to have disadvantages compared to solid forms, in terms of costs and stability.

Furthermore, the liquid form is an impediment to the formulation of a medicinal product 'in compositions with other active ingredients in a different physical state (see for example EP4316468A1, where the Serenoa extract is adsorbed on a solid silicon excipient to provide a solid formulation to be combined with other solid active ingredients). Phytochemical extracts are, however, highly complex chemical mixtures, therefore pre- and post-extraction treatments can have an impact on the final composition of the product; it is therefore important that any treatments do not significantly affect the composition of the extract and therefore the biological activity thereof.

The aim of the present invention is therefore to provide a product comprising an *Serenoa repens* extract as an active ingredient and excipients that are suitable to form a stable, homogeneous matrix, said composition also being suitable to be produced in solid form, with a high yield of dry product and no loss of biological activity.

### SUMMARY OF THE INVENTION

Said purpose has been achieved thanks to the composition according to the present invention, comprising a *Serenoa repens* lipid extract, lecithin, and pullulan, as stated in the claims.

In water, said composition forms a stable and homogeneous matrix in the form of an emulsion. Therefore, the present invention also concerns an emulsion comprising said composition and water. Furthermore, thanks to the chemical/physical characteristics of the composition according to the invention, said emulsion can be pulverised, preferably by spray drying, thereby obtaining a powder form of the Serenoa extract-based product without any reduction in the biological activity. Therefore, according to a further aspect, the present invention concerns the claimed composition in powder form, preferably obtainable by desiccation, more preferably by spray drying.

According to still further aspects, the present invention also concerns methods for producing the composition according to the invention in emulsion or powder form, as described in the claims, and the compositions thus obtained.

Surprisingly, the composition according to the invention not only maintains its biological activity even in emulsion or powder form, but has even improved biological activity compared to the *Serenoa repens* extract alone. Without wishing to be bound to the theory, it is therefore believed that the excipients of the composition, when formulated together with the active ingredient, contribute to the biological activity of the composition. According to still further aspects, the present invention is directed to the provision of a pharmaceutical formulation comprising the composition according to the invention and further pharmaceutically acceptable excipients, and also, optionally, further active ingredients. Preferably, said pharmaceutical formulation can be obtained by dissolving the composition according to the invention in said further excipients, preferably at a concentration of 1 to 5% weight/volume, more preferably of 2 to 4% weight/volume, and even more preferably of about 3% weight/volume (weight of the composition on the total volume of the pharmaceutical formulation).

In light of the known biological activities of *Serenoa repens,* which are maintained and even improved in the composition according to the present invention, according to still further aspects, the invention is also directed to the medical use of said composition, or formulations thereof, for the prevention and/or treatment of androgen-dependent disorders, such as benign prostatic hypertrophy or hyperplasia, prostate cancer, androgenetic alopecia, chronic pelvic pain, inflammation of the urinary tract, in particular of the bladder, diuresis disorders, decreased sexual desire, and hormonal imbalances. Particularly preferred are the medical uses of said composition, or of its formulations, for the prevention and/or treatment of benign prostatic hypertrophy or hyperplasia, prostate cancer, and androgenetic alopecia.

### BRIEF DESCRIPTION OF THE FIGURES

The characteristics and advantages of the present invention will become clear from the detailed description that follows, from the functional examples given for illustrative purposes, as well as from the annexed figures (Fig.), in which:
- Fig. 1 shows an emulsion obtained in Example 1.
- Fig. 2 shows (A) the powders obtained by spray drying the emulsions in Example 1 and (B) a detail of the powder obtained by spray drying the emulsions in Example 1.
- Fig. 3 shows the emulsion obtained by re-suspending in water powdered compositions of Serenoa extract, pullulan, and lecithin.
- Fig. 4 shows the fraction of total fatty acids in the powders of Example 2, obtained from emulsions prepared with lecithin at two different concentrations (0.5% or 1%) or without lecithin (0%).
- Fig. 5 shows the fatty acid profile after the spray drying of emulsions prepared with lecithin at two different concentrations (0.5% or 1%) or without lecithin (0%).
- Fig. 6 shows the fatty acid profile of a control sample containing only 1% lecithin, without Serenoa extract and pullulan and without spray drying.
- Fig. 7 shows the results of cell viability assays in human prostate carcinoma cells incubated for 24 hours (A) or 48 hours (B) with compositions according to the invention and comparative compositions, obtained by re-dissolving the powders in water at different serial dilutions.
- Fig. 8 shows the anti-androgenic effect of compositions according to the invention, obtained by re-dissolving in water the powders at different serial dilutions, compared with the Serenoa extract. The data is expressed as a % of AR production compared to the positive control treated with testosterone.
- Fig. 9 shows the absence of an anti-androgenic effect of the excipients of the compositions according to the invention when tested individually.

### DETAILED DESCRIPTION OF THE INVENTION

The composition according to the invention comprises:
a) *Serenoa repens* extract;
b) pullulan, and
c) lecithin.

*Serenoa repens* extract (*Serenoa repens* fruit extract, hereinafter also called Serenoa extract, for brevity) is an oily lipid extract, preferably obtained by hexane extraction (hexane extract), more preferably with a fatty acid titre of at least 85%, more preferably at least 85-95%.

Pullulan (CAS no. 9057-02-7) is a polysaccharide formed by repeating maltotriose units, connected to each other by an α-1,6 bond, isolated for the first time from a broth culture of the fungus *Aureobasidium pullulans.* Pullulan is an edible and non-toxic compound, which is usually used for its film-forming properties and to form resistant coatings for the production of tablets or capsules.

Lecithin is a well-known emulsifier, stabiliser, and preservative, which is also used in the food industry. For the purposes of the present invention, the lecithin may be soy or egg lecithin, preferably soy lecithin (CAS no. 8002-43-5); preferably, the lecithin is added to the composition in powder form.

According to preferred embodiments, the composition according to the invention comprises:
a) *Serenoa repens* extract and c) lecithin in a weight ratio (weight of Serenoa extract to weight of lecithin) of from 5:1 to 50:1, more preferably of from 10:1 to 25:1, even more preferably of from 10:1 to 15:1;
and/or b) pullulan and c) lecithin in a weight ratio (weight of pullulan to weight of lecithin) of from 5:1 to 10: 1;
and/or a) *Serenoa repens* extract and b) pullulan in a weight ratio (weight of Serenoa extract to weight of pullulan) of about 1: 1.

According to particularly preferred aspects, the composition according to the invention, comprising Serenoa extract, pullulan and lecithin, preferably at the weight ratios stated, further comprises water and it is in the form of an emulsion.

Said emulsion preferably comprises:
a concentration of a) *Serenoa repens* extract of about 3% to about 6%; more preferably of from about 4% to about 5%; and/or
a concentration of b) pullulan of from about 3% to about 6%; preferably of from about 4% to about 5%; and/or
a concentration of c) lecithin of from about 0.1% to about 1%, preferably from about 0.2% to about 0.8%, and more preferably from about 0.4% to about 0.6%; wherein said concentrations are weight/volume (w/v) concentrations based on the total volume of the emulsion.

Advantageously, the composition according to the invention forms a stable emulsion, allowing processing into a homogeneous phase.

In particular, said emulsion can be desiccated, preferably by spray drying, thereby obtaining a powder composition, capable of reforming a stable, homogeneous emulsion once re-suspended in water. According to preferred aspects then, the composition according to the invention, comprising Serenoa extract, pullulan, and lecithin, preferably in the stated weight ratios, is a powder and more preferably is obtainable by desiccating the emulsified composition. Such emulsions can be desiccated to a powder, with a high yield.

More preferably the emulsion composition is an aqueous solution comprising:
a) *Serenoa repens* extract;
b) pullulan, and
c) lecithin.
wherein the weight ratio of a) *Serenoa repens* extract to c) lecithin is from 5:1 to 10:1, the weight ratio of b) pullulan to c) lecithin is from 5:1 to 10:1, and the weight ratio of a) *Serenoa repens* extract to b) pullulan is approximately 1:1.

After desiccation, this composition can provide a yield of powdered product ranging from 20% to 50% (calculated as a percentage of the weight of the dry product obtained with respect to the theoretical weight of the dry product) on a laboratory scale.

Even more preferably, the emulsion composition is an aqueous solution comprising:
a) *Serenoa repens* extract;
b) pullulan, and
c) lecithin.
wherein the weight ratio of a) *Serenoa repens* extract to c) lecithin is approximately 5:1, the weight ratio of b) pullulan to c) lecithin is approximately 5:1 to 10:1, and the weight ratio of a) *Serenoa repens* extract to b) pullulan is approximately 1:1.

After desiccation, this composition can provide a yield of powdered product ranging from 35% to 50% (calculated as a percentage of the weight of the dry product obtained with respect to the theoretical weight of the dry product) on a laboratory scale.

These yields are considered predictive of yields of up to 90% on an industrial scale, when the processing volumes are much higher.

Advantageously, furthermore, the composition according to the invention allows a powder composition to be obtained with a very high yield of fatty acids, which are the bioactive components of the Serenoa extract.

Particularly preferred are the aqueous emulsions obtained from the matrix shown in Table 1 below:

**Table 1 *weight concentration based on the final emulsion volume**

| | Serenoa extract (w/v) * | Pullulan (w/v) * | Lecithin (w/v) * |
|---|---|---|---|
| Product 1 | 5% | 5% | 0.1% |
| Product 2 | 5% | 5% | 0,5% |
| Product 3 | 5% | 5% | 1% |

In some embodiments, the composition according to the invention consists essentially of a) *Serenoa repens* extract, b) pullulan, and c) lecithin. The expression "consists essentially of" means that *Serenoa repens* extract, pullulan, and lecithin are the only ingredients in the composition that are essential to the biological activity while any further component or excipient do not interfere with their action. It should also be understood that all the aspects identified above as preferred and advantageous for the composition and the components thereof should be deemed equally preferred and advantageous also for these embodiments.

In further embodiments, the composition according to the invention consists of a) *Serenoa repens* extract, b) pullulan, and c) lecithin, and optionally water. It should also be understood that all the aspects identified above as preferred and advantageous for the composition and the components thereof should be deemed equally preferred and advantageous also for these embodiments.

Particularly preferred are compositions in which the weight ratio of a) *Serenoa repens* extract to c) lecithin is from 50:1 to 5:1, more preferably from 10:1 to 5:1, and the weight ratio of b) pullulan to c) lecithin is from 50:1 to 5:1, more preferably from 10:1 to 5:1 and/or the weight ratio of a) *Serenoa repens* extract to b) pullulan is approximately 1:1.

Further aspects of the invention include methods for producing the composition according to the invention in emulsion form or in powder form, comprising:
i. mixing a mixture of the following components in water: a) the *Serenoa repens* extract and c) lecithin, thereby obtaining a base emulsion;
ii. adding an aqueous solution of b) pullulan and water to the base emulsion, and emulsifying, optionally using a homogeniser, thereby obtaining the composition in emulsion form; and
iii. optionally desiccating the emulsion, preferably by spray drying, thereby obtaining the powder composition.

According to further aspects, the present invention is also directed to the production of pharmaceutical formulations comprising the composition of *Serenoa repens* extract, pullulan and lecithin, and further pharmaceutically acceptable excipients.

The term "excipient" herein means any substance, other than an active agent, that can be used as a carrier or vehicle for administration of an active agent to a subject, or combined with an active agent to improve the handling or storage properties thereof, or to enable or facilitate formation of a dosage unit of the composition.

Pharmaceutically acceptable excipients may be rheological additives, buffering agents, antioxidant agents, anti-isothermal agents, antistatic agents, absorbent agents, chelating agents, preservative agents, denaturing agents, additional emulsifying agents, plasticising agents, propellant agents, reducing agents, solvents, additional stabilising agents, or mixtures thereof.

In accordance with preferred embodiments, the pharmaceutical product according to the invention comprises a pH regulating agent, preferably in a sufficient amount for the composition to maintain a pH of 5-7.

Optionally, said pharmaceutical formulations may include further active ingredients.

Indeed, advantageously, the composition according to the invention, potentially as a stable, biologically active emulsion or powder, can easily be mixed with further active ingredients, regardless of the physical form of such further active ingredients. Preferably, pharmaceutical formulations according to the present invention are obtainable by dissolving in said further excipients the composition according to the invention, at such a concentration as to obtain a final composition concentration of between 1 and 5% weight/volume, more preferably between 2 and 4% weight/volume, and even more preferably approximately 3% weight/volume, based on the total volume of the pharmaceutical formulation, said formulation optionally also comprising further active ingredients.

The composition according to the invention has been shown to have anti-androgenic activity. This activity is maintained following processing of the composition, for example after emulsion and after desiccation.

Surprisingly, this anti-androgenic activity has been shown to be even higher than that of the Serenoa extract alone.

Therefore, according to a further aspect, the invention is directed to the production of the composition according to the invention in any of the forms described herein, or in any formulations thereof, for use in the prevention and/or treatment of androgen-dependent disorders. Preferably, said disorders include: benign prostatic hypertrophy or hyperplasia, prostate cancer, androgenetic alopecia, diuresis disorders, decreased sexual desire, and hormonal imbalances. Furthermore, given the known anti-inflammatory activity of the Serenoa extract, particularly in the urogenital tract, said disorders also include: chronic pelvic pain and inflammation of the urinary tract, particularly of the bladder.

The term "prevention and/or treatment" refers to the effects of the composition according to the invention, which can provide a benefit to patients affected by these disorders, for example by improving the patient's condition or by delaying progression of the disorder. Preferably, the composition according to the invention, and the forms and formulations thereof, must be administered orally.

Preferably, this composition should be administered in a dose such as to provide a supply of Serenoa extract amounting to between 100 and 600 mg per day, more preferably from 160 to 320 mg per day, said composition having a fatty acid thiol of not less than 20% by weight, based on the weight of the composition, the effective dosage being dependent on the extent and severity of the disease to be treated.

It is understood that all the aspects identified as preferred and advantageous for the composition according to the invention are to be considered equally preferred and advantageous also for the pharmaceutical formulation and uses thereof.

The following embodiments of the present invention are provided as non-limiting illustrative examples.

### EXAMPLES

### Example 1

An oily Serenoa lipid extract, with 85-95% fatty acid content, was supplemented with pullulan or cyclodextrin together with two different concentrations of lecithin emulsifier to form an emulsion to be subsequently desiccated by spray drying.

The compositions prepared according to the matrix in Table 2, were suspended in water and mixed for approximately one minute with a homogeniser.

**Table 2 * concentration by weight based on the final volume of the emulsion**

| | Serenoa extract (w/v) * | Pullulan (w/v) * | Cyclodextrin (w/v) * | Lecithin (w/v) * |
|---|---|---|---|---|
| Product 1 | 5% | 5% | -- | 0.5% |
| Product 2 | 5% | 5% | -- | 1% |
| Product 3 (comparative) | 5% | -- | 5% | 0,5% |
| Product 4 (comparative) | 5% | -- | 5% | 1% |

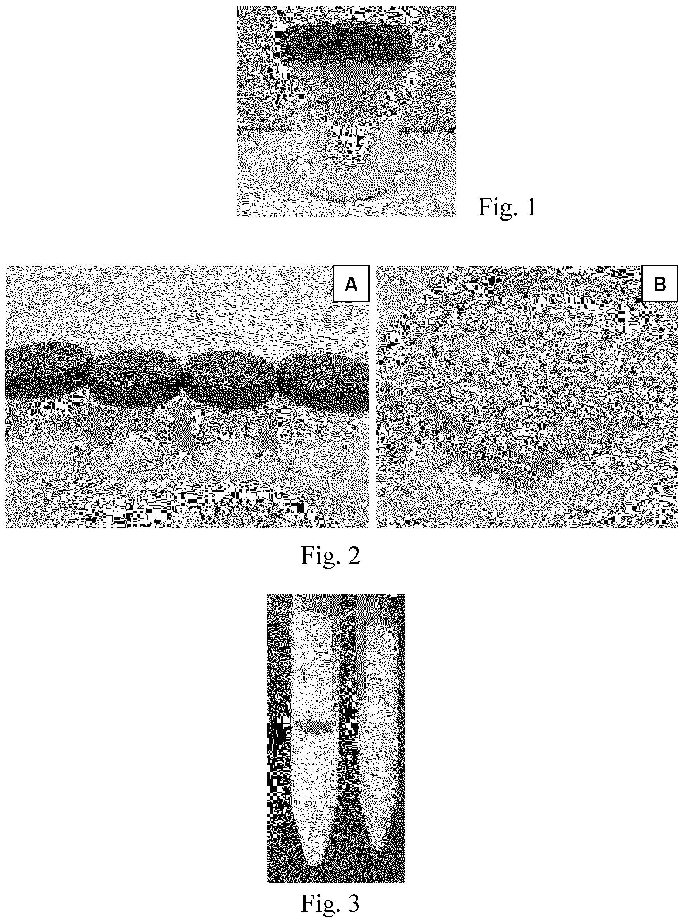

Figure 1 shows, by way of example, one of the emulsions obtained.

The emulsions prepared were desiccated by spray drying using an inlet temperature of 150 °C. The tests were performed in a Buchi B-290 Laboratory Spray Dryer.

At the end of the desiccation stage, the powders (shown in Fig. 2) were then collected, quantified, and analysed.

The yields obtained after spray drying are shown in Table 3.

**Table 3**

| | Processed volume | Theoretical dry weight | Actual dry weight | Yield (%) |
|---|---|---|---|---|
| Product 1 | 50 ml | 5.25 g | 1.45 g | 27.6 % |
| Product 2 | 50 ml | 5.5 g | 2.59 g | 47.0 % |
| Product 3 (comparative) | 50 ml | 31.5 g | 1.79 g | 5.6 % |
| Product 4 (comparative) | 50 ml | 33 g | 3.05 g | 9.2 % |

Surprisingly, the yields obtained in the presence of pullulan are 3 to 8 times higher than the yields obtained in the presence of cyclodextrin.

The powders obtained after spray drying were then tested to verify whether the emulsion was maintained once re-suspended in water.

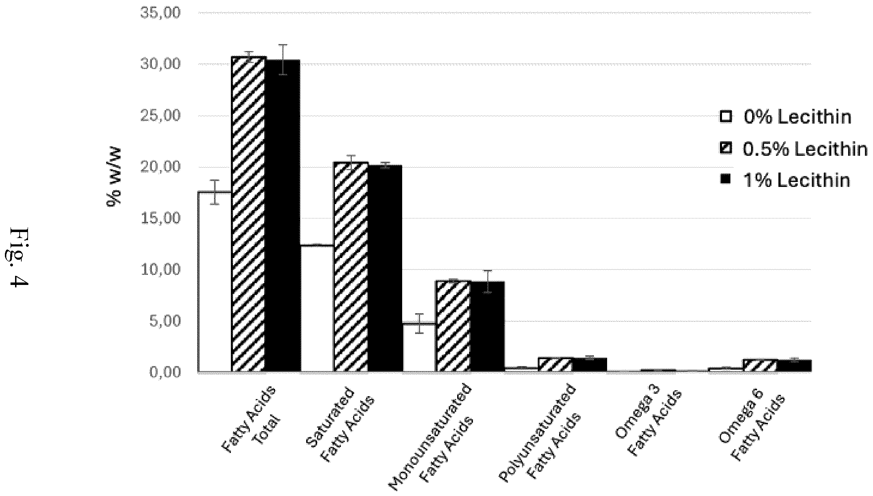

Figure 4 shows the powders of the products with the best yield (Products 1 and 2, according to the invention), re-suspended in water at 3% w/v: restoration of the stable emulsion is visible in both products.

The oily extract supplemented with pullulan and lecithin forms a stable emulsion, treatable by spray drying to obtain a solid product. In particular, the presence of lecithin prevented coalescence of the oil micelles, thereby postponing phase separation. This facilitates protection thereof during the spray drying process.

From the desiccation tests it was also observed that the recovery yields increase as the lecithin content increases, with a much higher yield in the presence of pullulan compared with cyclodextrin.

All the emulsions prepared can be desiccated by spray drying, thereby obtaining a solid product composed of a dehydrated powder.

Solubilisation tests conducted on the powders obtained provided very positive results, confirming the effectiveness of the invention to obtain a desiccated powder, which returns to a homogeneous, stable emulsion after re-suspension in water.

The composition according to the invention therefore allows formulation in both solid form (in the form of powder) and with an aqueous matrix (in the form of emulsion).

### Example 2

An oily Serenoa lipid extract, with a fatty acid content of 85-95%, was supplemented with pullulan and lecithin.

The emulsions to be spray dried were prepared according to the mass balance shown in the following diagram.

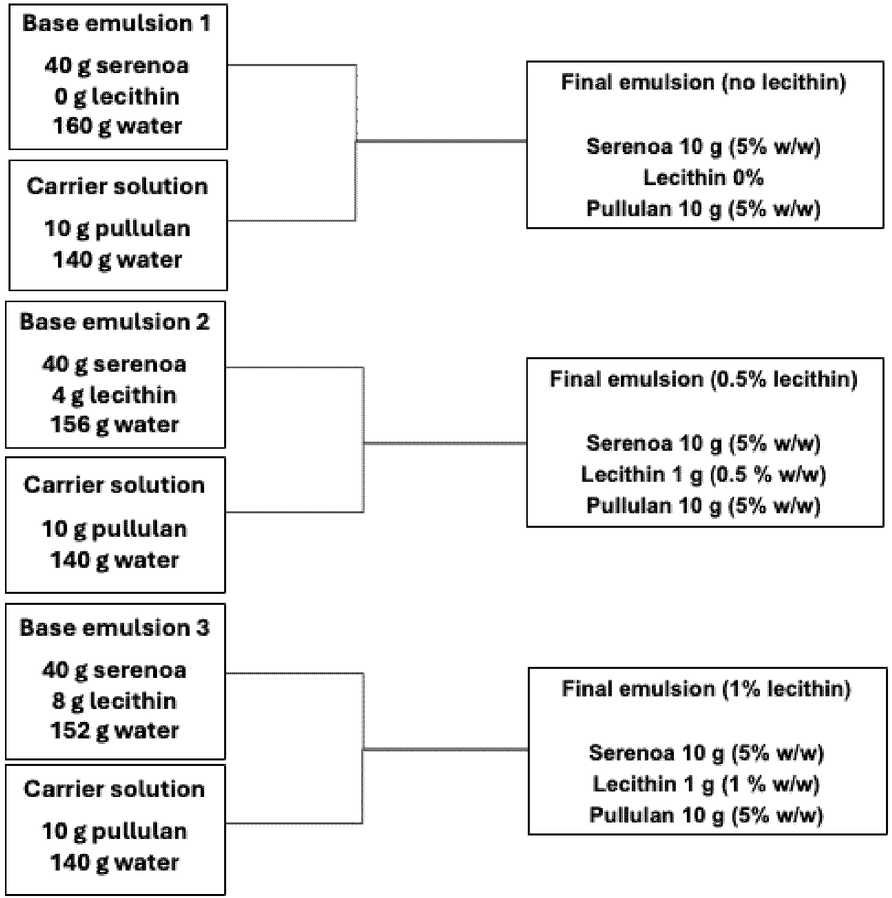

Each base emulsion was produced by mixing the required amount of Serenoa extract, optional soy lecithin (Millipore), and water.

Stirring was applied at 20000 rpm in three 20-second bursts using a 170 W Turrax IKA with a 13 mm stator and 4 mm orifices.

50 g of each base emulsion was dispersed under constant stirring with a magnetic bar, in 150 g of carrier solution consisting of water and pullulan (Hayashibara) resulting in 200 g of final emulsion. The emulsions were prepared in duplicate.

The emulsions prepared (3 final emulsions, in duplicate) were desiccated using a Buchi B-290 Laboratory Spray Dryer, set as follows: inlet temperature: 150 °C, flow rate: 3% (0.9 ml min-1), air flow rate: 0.667 m³h⁻¹, and intake: 100% (approximately 35 m³ h⁻¹). Following completion of the desiccation phase, the powders were collected, quantified, and analysed.

All samples gave rise to the formation of dry solids.

The sample without lecithin failed to form a stable emulsion. The preparation formed two phases in approximately one hour, requiring constant stirring to stabilise it during spray drying. This was not observed in the samples containing soy lecithin, whose emulsions remained stable for days.

The yields obtained are summarised in Table 4 (each emulsion comprising 5% Serenoa extract, 5% pullulan, and the lecithin concentrations stated).

**Table 4**

| Final emulsions | Average yield (%) | Standard dev. |
|---|---|---|
| With 0% lecithin | 2.75 | 0.07 |
| With 0.5% lecithin | 25.26 | 0.11 |
| With 1% lecithin | 24.48 | 2.88 |

The presence of lecithin helped facilitate the spray drying process, resulting in average yields of 25.26% and 24.48% for the samples with 0.5% and 1% soy lecithin, respectively.

### Example 3

The solids obtained in Example 2 were re-suspended in water for gas chromatographic (GC) analysis.

GC analysis showed a much higher percentage of fatty acids in the solids recovered from the samples containing 0.5% and 1% lecithin in the emulsion, almost double that found in the samples without soy lecithin (Fig. 4).

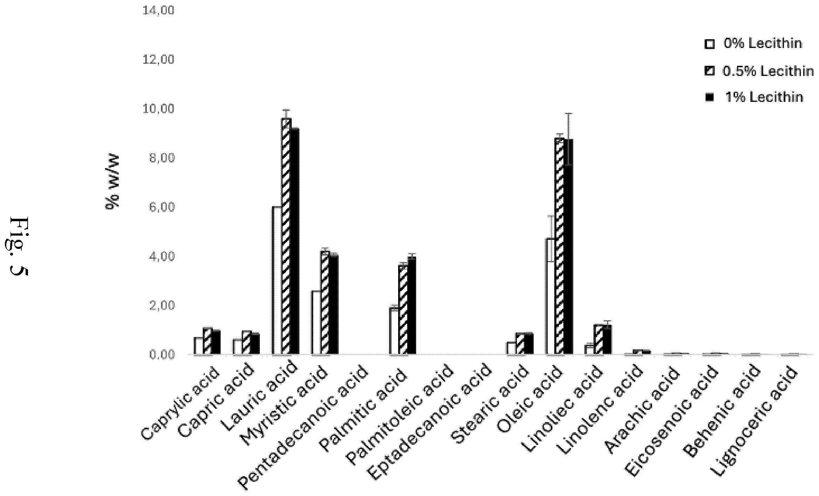

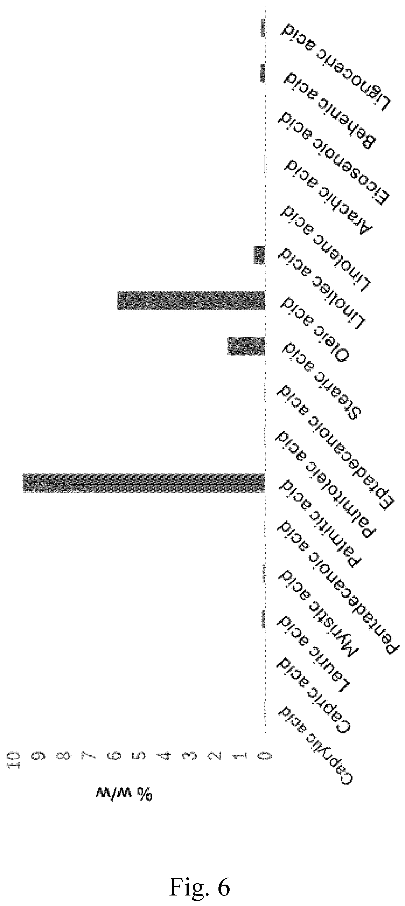

A fatty acid profile was determined using GC, as shown in Fig. 5: lauric acid and myristic acid are the predominant fatty acids in the Serenoa lipid extract, while these acids are very low in soy lecithin, as evidenced in a control sample containing only 1% lecithin and no Serenoa extract or pullulan (Fig. 6), not subjected to spray drying. This data demonstrates that the emulsions containing 0.5% and 1% lecithin led to a higher proportion of fatty acids from the Serenoa extract in the end product.

In particular, high proportions of key fatty acids, such as lauric acid, myristic, palmitic, oleic and linolenic, were found in the samples according to the invention.

### Example 4

Emulsions were produced from compositions comprising Serenoa extract, lecithin, and pullulan according to the invention or comparative compositions comprising Serenoa extract, lecithin, and cyclodextrin, as described in Example 1 (products 1 to 4 in Table 2); the emulsions were then desiccated by spray drying and subsequently re-dissolved in aqueous solution at 3% w/w (composition weight with respect to the total weight of the aqueous solution), thereby obtaining the respective four formulations F1, F2, F3, and F4 further diluted to obtain the scalar concentrations used in the cell viability assays.

The cell viability assay was conducted in order to identify non-toxic compositions *in vitro.*

The experiments were performed on a human androgen-dependent prostate cancer cell line, similar to a prostatic hypertrophy condition (LnCaP cells). The cells were grown at 37 °C, 5% CO² in RPMI1640 medium, supplemented with 1% L-Glutamine and 10% FBS.

The LnCaP cells were plated in 96-well plates at a density of 10⁴ cells/well, in growth medium supplemented with 10% FBS.

The following day, the medium was removed and replaced with 100 µL of complete medium supplemented with 1% FBS and the treatment was performed with the four formulations, and with the Serenoa extract as-is as reference.

In particular, the cells were treated with 10 scalar concentrations of each formulation and of Serenoa as is, obtained by serial 1:2 dilutions, corresponding to the following concentrations of active ingredient (Serenoa):
1 - 0.5 - 0.25 - 0.125 - 0.063 - 0.031 - 0.016 - 0.008 - 0.004 - 0.002 mg/ml Sodium dodecyl sulfate (SDS) was used as a positive control of toxicity at a concentration of 1 mg/ml.

The cells were incubated for 24 or 48 hours at 37 °C and 5% CO₂. At the end of each incubation, 3-(4,5-dimethylthiazol-2-yl)-2,5-diphenyltetrazolium bromide (MTT) reagent was added to each well at a final concentration of 0.5 mg/ml and the plates were incubated for another 2 hours at 37 °C and 5% CO₂.

At the end of the incubation, the reduced MTT crystals were solubilised by removing the medium and adding 100 µl of dimethyl sulfoxide (DMSO) to each well. Finally, absorbance at 595 nm of each sample was measured using an Infinite M NANOP plate reader (Tecan).

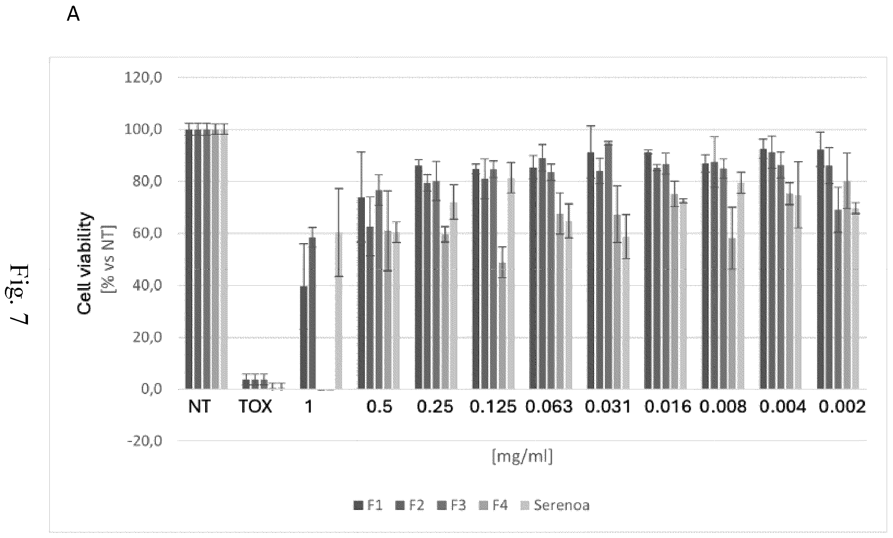

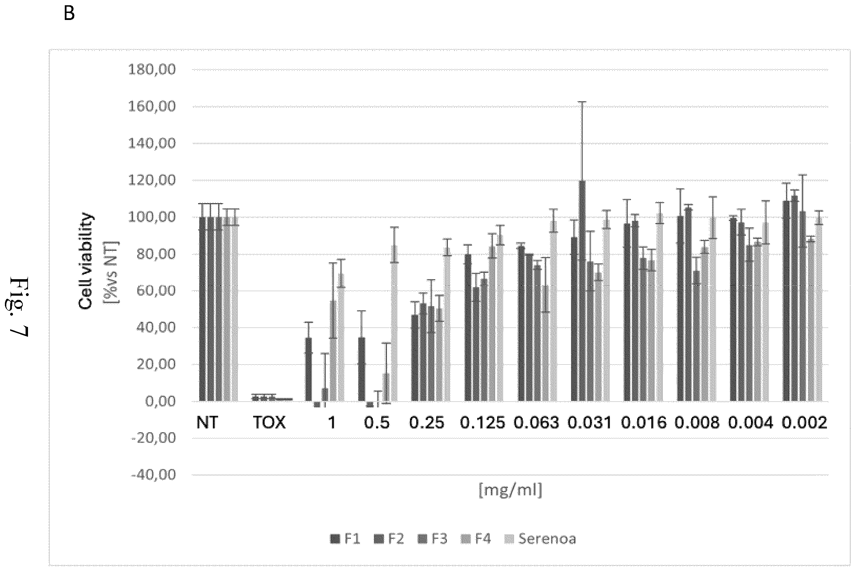

Cell viability after 24 (A) or 48 hours (B) of treatment is shown in Figure 7; viability is expressed as a percentage of the absorbance of the untreated cells (UT) at 595 nm.

The percentage averages are also shown in Table 6 (at 24 hours, A, and at 48 hours, B).

**Table 6A**

| | **UT** | **TOX** | **(mg/ml active)** | | | | | | | | | |
|---|---|---|---|---|---|---|---|---|---|---|---|---|
| | | | **1** | **0.5** | **0.25** | **0.125** | **0.063** | **0.031** | **0.016** | **0.008** | **0.004** | **0.002** |
| **F1** | 100.0 | 3.7 | 39.6 | 73.8 | 86.1 | 84.8 | 85.4 | 91.2 | 91.3 | 86.8 | 92.6 | 92.3 |
| **F2** | 100.0 | 3.7 | 58.4 | 62.6 | 79.3 | 80.9 | 89.0 | 83.9 | 85.3 | 87.4 | 91.3 | 86.1 |
| **F3** | 100.0 | 3.7 | -2.3 | 76.6 | 80.1 | 84.6 | 83.5 | 94.6 | 86.7 | 84.9 | 86.2 | 69.0 |
| **F4** | 100.0 | 0.6 | -3.4 | 60.9 | 59.5 | 48.8 | 67.6 | 67.3 | 75.0 | 58.0 | 75.2 | 80.1 |
| **Serenoa** | 100.0 | 0.7 | 60.2 | 60.3 | 71.9 | 81.4 | 64.7 | 58.6 | 72.4 | 79.4 | 74.8 | 69.6 |

**Table 6B**

| | **UT** | **TOX** | **(mg/ml active)** | | | | | | | | | |
|---|---|---|---|---|---|---|---|---|---|---|---|---|
| | | | **1** | **0.5** | **0.25** | **0.125** | **0.063** | **0.031** | **0.016** | **0.008** | **0.004** | **0.002** |
| **F1** | 100.0 | 2.8 | 34.5 | 34.6 | 46.8 | 79.9 | 84.6 | 89.0 | 96.6 | 100.5 | 99.4 | 108.9 |
| **F2** | 100.0 | 2.8 | -4.7 | -3.3 | 53.1 | 61.9 | 79.7 | 119.7 | 97.9 | 105.2 | 97.2 | 111.6 |
| **F3** | 100.0 | 2.8 | 7.3 | -0.1 | 51.7 | 66.6 | 74.1 | 76.0 | 77.9 | 71.1 | 85.0 | 103.3 |
| **F4** | 100.0 | 1.2 | 54.7 | 15.3 | 50.4 | 84.4 | 63.1 | 70.2 | 76.8 | 83.9 | 86.6 | 88.1 |
| **Serenoa** | 100.0 | 1.2 | 69.5 | 85.0 | 83.7 | 90.3 | 98.0 | 98.7 | 102.2 | 99.6 | 97.1 | 99.6 |

After 24 hours of treatment, at all the concentrations assayed:
the as-is Serenoa extract determines a cell viability greater than or equal to 60%, although only one experimental group, corresponding to the treatment with 0.125 mg/ml, showed a cell viability (slightly) above 80%;
treatment with 0.5 mg/ml of Formulation 1, containing 0.5% lecithin and 5% pullulan, determined an average cell viability of 73.8%, while at all subsequent concentrations, the cell viability was always above 80%;
Formulation 2, containing 1% lecithin and 5% pullulan, determined a cell viability of around 60% when the cells were treated with the highest two concentrations in the assay (1 - 0.5 mg/ml). At all subsequent concentrations, cell viability was always greater than or equal to 80%;
Formulation 3, containing 0.5% lecithin and 5% cyclodextrin, determined a high toxicity when the cells were treated with the highest concentration in the assay (1 mg/ml); Formulation 4, containing 1% lecithin and 5% cyclodextrin, determined high toxicity when the cells are treated with the maximum concentration in the assay (1 mg/ml); at none of the subsequent concentrations assayed was the cell viability higher than 80% since all subsequent results were between 50 (0.125 mg/ml) and 80% (0.002 mg/ml). The formulations according to the invention, including pullulan, in addition to lecithin, are therefore safer than the formulations with cyclodextrin.

After 48 hours of treatment, at all the concentrations assayed:
Serenoa as is determined a cell viability greater than or equal to 70%: starting from the concentration 0.125 mg/ml, the viability was always greater than 90%;
Formulation 1, containing 0.5% lecithin and 5% pullulan, determined a cell viability of less than 50% when the cells were treated with the three maximum concentrations in the assay (1 - 0.5 - 0.25 mg/ml); all subsequent concentrations determined a cell viability that was always greater than or equal to 80%.
Formulation 2, containing 1% lecithin and 5% pullulan, had a viability of between 50 and 60% when treated with 0.25 and 0.125 mg/ml and a viability greater than or equal to 80% at all subsequent concentrations.
Formulation 3, containing 0.5% lecithin and 5% cyclodextrin, determined high toxicity when the cells were treated with the highest two concentrations in the assay (1 and 0.5 mg/ml), a viability of between 50 and 70% when they were treated with 0.25 and 0.125 mg/ml, and a viability greater than or equal to 70% at all subsequent concentrations; Formulation 4, containing 1% lecithin and 5% cyclodextrin, had a cell viability greater than 60% starting with the concentration of 0.125 mg/ml.

### Example 5

In order to verify anti-androgenic activity of the formulations, the expression of the androgen receptor in androgen-dependent prostatic cells was used as a surrogate marker. The androgen receptor (AR) is a nuclear receptor that is physiologically present at intracellular level, in cytoplasm, in an inactivated state. To exert their action, androgens must therefore diffuse through cell membranes, in order to reach the cytoplasmic receptor molecules, bind thereto, and induce the nuclear translocation thereof. At this point, the AR can bind the DNA promoter and regulate the expression of androgen-dependent genes, thereby activating specific signalling pathways (e.g. stimulation of cell proliferation). The level of AR production therefore represents a determining factor for androgens to exert their action.

LnCaP cells were plated in 48-well plates at a density of 5×10⁴ cells/ml, in medium with 10% FBS. After 24 hours, the medium was replaced with 500 µL of complete medium without FBS, and at the same time a state of hyperandrogenism was induced by treatment with Testosterone (T) 10⁻⁹M in the presence of the products to be assayed. In particular, based on the previous cell viability assay, the three concentrations of active ingredient to be used for the assay were selected: 0.125 - 0.063 - 0.031 mg/ml.

An experimental group of untreated cells was included as a "normal" hormonal control, as was an experimental group of cells treated only with Testosterone as a "hypertrophic" condition. The treatments were maintained for 24 or 48 hours: upon cell harvesting, the total intracellular proteins were extracted using a lysis buffer, and the AR was measured using a semi-quantitative ELISA, following the kit protocol provided by the manufacturer.

The assay results were processed also taking into account cell viability, to ensure the AR reductions observed were actually due to lower AR expression and not to lower cell viability/fewer cells; expression data was then normalised with respect to the viability % of each experimental group.

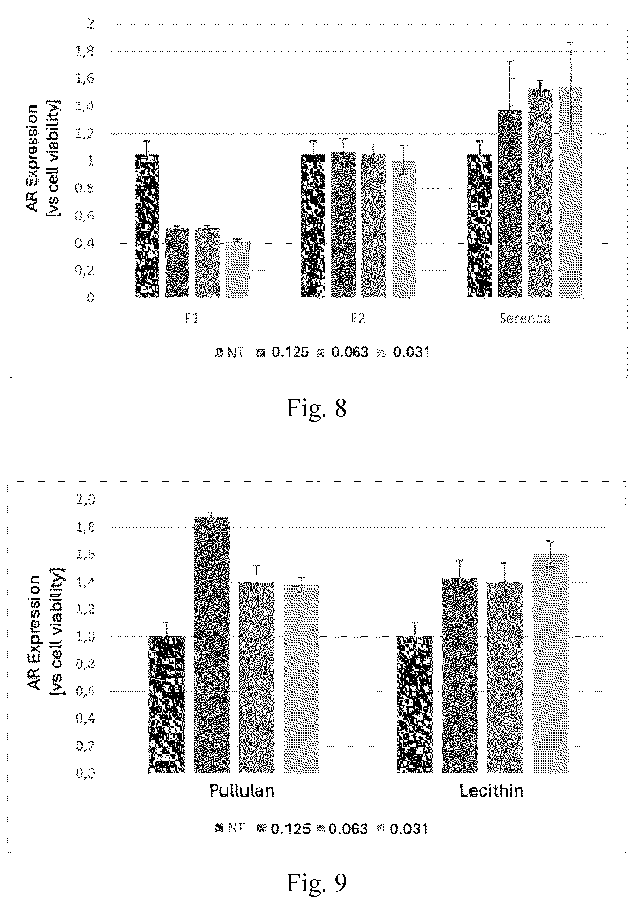

The test results are shown in Fig. 8.

The as-is Serenoa extract even induced an increase in AR production as the concentration of said Serenoa decreases. This effect was not observed in Formulation 2 (comprising pullulan and 1% lecithin as excipients) and a reduction in AR expression was advantageously observed after treatment with Formulation 1 (comprising pullulan and 0.5% lecithin).

For the sake of completeness, the production of androgen receptors was also analysed following treatment with solely the as-is excipients (lecithin and pullulan), in order to determine whether these substances alone could have contributed to the reduction of the AR. The results obtained are shown in Figure 9, where it can be seen that the excipients did not directly reduce AR production.

## Claims

1. Composition comprising:
a) extract of *Serenoa repens;*
b) pullulan, and
c) lecithin.

2. The composition of claim 1, wherein a) the extract of *Serenoa repens* is a hexane extract.

3. The composition of claim 1 or 2, wherein a) the extract of *Serenoa repens* has a fatty acids titre of at least 85%, preferably of 85-95%.

4. The composition of any one of claims 1 to 3, wherein:
the weight ratio of a) *Serenoa repens* extract and c) lecithin is from 5:1 to 50:1;
and/or the weight ratio of b) pullulan and c) lecithin is from 5:1 to 50:1; and/or the weight ratio of a) *Serenoa repens* extract and b) pullulan is approximately 1:1.

5. The composition of any one of claims 1 to 4, wherein:
the weight ratio of a) *Serenoa repens* extract and c) lecithin is from 10:1 to 25:1,
preferably from 10:1 to 15:1; and/or the weight ratio of b) pullulan and c) lecithin is from 10:1 to 25:1, preferably from 10:1 to 15:1; and/or the weight ratio of a) *Serenoa repens* extract and b) pullulan is approximately 1:1.

6. The composition of any one of claims 1 to 5 in the form of an emulsion, further comprising water.

7. The composition of claim 6, comprising:
a) *Serenoa repens extract* at a concentration by weight of from about 3% to about 6%, preferably from about 4% to about 5%; and/or
b) pullulan at a concentration by weight of from about 3% to about 6%, preferably from about 4% to about 5%; and/or
c) lecithin at a concentration by weight of from about 0.1% to about 1%, preferably from about 0.2% to about 0.8%, more preferably from about 0.4% to about 0.6%,
on the total weight of the emulsion.

8. The composition of any one of claims 1 to 7, in powder form, preferably obtainable by drying the composition of any one of claims 6 or 7.

9. Method for producing the composition of any one of claims 6 or 7, comprising:
i. mix in water a mixture comprising: a) the extract of *Serenoa repens,* and c) lecithin, obtaining a base emulsion; and
ii. add to the base emulsion an aqueous solution of b) pullulan and water, and emulsify, optionally using a homogenizer, obtaining the composition in the form of an emulsion.

10. Method for producing the composition of claim 8, comprising:
i. mix in water a mixture comprising: a) the extract of *Serenoa repens,* and c) lecithin, obtaining a base emulsion;
ii. add to the base emulsion an aqueous solution of b) pullulan and water, and emulsify, optionally using a homogenizer, obtaining a composition in the form of an emulsion; and
iii. dry the emulsion, preferably by spray drying, obtaining the composition in powder form.

11. Pharmaceutical formulation comprising the composition of any one of claims 1 to 8, and further pharmaceutically acceptable excipients, and optionally further active ingredients.

12. The pharmaceutical formulation of claim 11, obtainable by dissolving the composition of claim 8 in said further excipients at a concentration between 1 and 5% weight/volume, preferably between 2 and 4% weight/volume, more preferably of about 3% weight/volume, based on the total volume of the pharmaceutical formulation.

13. The composition of any one of claims 1 to 8, or the formulation of any one of claims 11 or 12, for use as a medicament.

14. The composition of any one of claims 1 to 8, or the formulation of any one of claims 11 or 12, for use in the prevention and/or treatment of androgen-dependent disorders, such as benign prostatic hypertrophy or hyperplasia, prostate cancer, androgenetic alopecia, chronic pelvic pain, inflammation of the urinary tract, particularly of the bladder, diuresis disorders, decreased sexual desire, hormonal imbalances.

15. The composition or formulation for the use of claims 13 or 14, said composition or formulation being obtained by the method of claim 9 or 10.
